# EUROPÄISCHE PATENTSCHRIFT

(11) **EP 0 923 721 B1**
(45) Veröffentlichungstag und Bekanntmachung des Hinweises auf die Patenterteilung: **05.11.2003**
(21) Anmeldenummer: 97939951.6
(22) Anmeldetag: 12.08.1997
(51) Int. Cl.: G01N 27/02

(54) **SENSOR ZUR ÜBERWACHUNG EINES NOx-KATALYSATORS**
MONITORING SENSOR FOR NOx CATALYTIC CONVERTER
CAPTEUR POUR LA SURVEILLANCE D'UN CATALYSEUR DE REDUCTION DES NOx

(30) Priorität: 05.09.1996 DE 19635977
(43) Veröffentlichungstag der Anmeldung: 23.06.1999
(73) Patentinhaber: ROBERT BOSCH GMBH, 70442 Stuttgart (DE)
(72) Erfinder: SEIPLER, Dieter, D-72766 Reutlingen (DE); SCHUMANN, Bernd, D-71277 Rutesheim (DE)
(86) Internationale Anmeldenummer: DE9701714
(87) Internationale Veröffentlichungsnummer: WO98010272

(56) Entgegenhaltungen:
- EP-A- 0 560 991
- US-A- 5 369 956
- US-A- 5 546 004

## Beschreibung

Die Erfindung geht aus von einem Sensor zur Überwachung eines NOx-Katalysators nach der Gattung des Hauptanspruches. Der konventionelle Drei- Wege- Katalysator zur Abgasreinigung von Verbrennungsmotoren entfernt in sehr effizienter Weise die Abgaskomponenten HC, CO und NOx, allerdings nur unter der während der im Fahrzyklus überwiegenden Voraussetzung, dass die Abgaszusammensetzung nahe beim stöchiometrischen Luft/Kraftstoff- Wert liegt. Für oxidierte Abgase, wie z.B. von Mager - und Dieselmotoren hat sich der NOx-Adsorptions/- Reduktions- Katalysator durchgesetzt, der nach dem folgenden Prinzip arbeitet:

Während der im Fahrzyklus überwiegenden Phase mageren Abgasausstoßes wird NOx im Adsorbermaterial gespeichert, um dann einem Fettimpuls desorbiert und reduziert zu werden. Ein derartiger NOx-Katalysator ist z.B. in der EP-A-0 560 991 beschrieben. Darin werden als Adsorberstoffe Alkalimetalle, Erdalkalimetalle, Seltenerdmetalle sowie Edelmetalle genannt, die auf einem Trägerstoff wie z.B. Aluminiumoxid aufgebracht sind. Als voraussichtlicher Reaktionsmechanismus wird in der Magerphase die Adsorption von Sauerstoff in Form von O₂genannt, der mit dem NOx aus dem Abgas zu NO2 reagiert. Ein Teil des gebildeten NO2 wird weiter oxidiert und diffundiert in den Adsorberstoff in Form von Nitrationen. Bei einer Fettverschiebung des Abgaszusammensetzung findet die obengenannte Reaktionsfolge in umgekehrter Richtung statt, d. h. NOx wird mit den reduzierten Komponenten HC und CO des fetteren Abgases zu freiem Stickstoff reduziert. Der Katalysator arbeitet überwiegend in der Magerphase, das zeitliche Verhältnis von der Magerphase zu Fettimpuls beträgt etwa 50:1.

Ein wesentliches Problem ist es, zu erkennen, wann die Speicherkapazität des Adsorberstoffes erschöpft ist und der Fettimpuls gesetzt werden muss. Dazu wird gemäß der EP-A-0 560 991 die kumulative Fahrzeuggeschwindigkeit gemessen und daraus der Beladungsgrad des Adsorberstoffes geschätzt. Die genaue Kenntnis des Beladungsgrades ist nach dieser Methode nicht möglich.

In der US 5,546,004 ist ein Sensor für Abgasnachbehandlungssysteme beschrieben, der der Bestimmung der Konzentration einer absorbierten Substanz, insbesondere Ammoniak, dient.

Dieser weist ein Ammoniak absorbierendes Material in Form von Titandioxid in Mischung mit einem Wolframoxid, Molybdänoxid oder Vanadiumpentoxid auf. Dabei handelt es sich um Materialien zur katalytischen Zersetzung von Stickoxiden (De-NOx-Materialien), die für die Absorption von Ammoniak bei SCR-Katalysatoren bekannt sind und Ammoniak besonders gut binden.

Auch der US 5,396,956 ist ein Abgasnachbehandlungssystem zu entnehmen, das mindestens einen Ammoniaksensor aufweist. Einer dieser Ammoniaksensoren kann beispielsweise im Trägermaterial eines Abgaskatalysators vorgesehen sein. Diese Sensoren sind zur Kontrolle eines NOx-Speicherkatalysators nur bedingt geeignet.

Demgegenüber liegt der vorliegenden Erfindung, wie sie im Anspruch 1 definiert ist, die Aufgabe zugrunde, einen Sensor zur Überwachung eines NOx-Speicherkatalysators bereitzustellen, mit dem der aktuelle Beladungsgrad des Speichermaterials bestimmt werden kann.

Dadurch, daß der Beladungsgrad des Speichermaterials genau überwacht wird, kann die Speicherkapazität besser ausgenutzt und der Katalysator kostengünstiger dimensioniert werden. Die Konversionsphase der Anfettung kann präziser vorhergesagt und damit im Fahrzyklus günstiger untergebracht werden.

Indem das Speichermaterial des Katalysators zugleich als Basiselement für den Sensor zur Beladungsmessung fungiert, wird eine raum- und kostensparende Lösung gegenüber einer Nachkatalysatorsonde erreicht.

Durch die in den Unteransprüchen aufgeführten Maßnahmen sind vorteilhafte Weiterbildungen und Verbesserungen des im Hauptanspruch angegebenen Sensors möglich.

In günstiger Weise wird der Sensor so aufgebaut, daß er auf eine elektrische oder elektromagnetische Eigenschaft des Speichermaterials anspricht, die sich mit der adsorbierten NOx- Menge ändert. Besonders einfach kann der Sensor als Kondensator ausgebildet sein, dessen Kapazität sich mit der Dielektrizitätskonstante ändert, die ihrerseits vom NOx- Beladungsgrad des Speichermaterials abhängt.

In vorteilhafter Weise kann der Sensor auch als Resonator, Hohlraumresonator oder gefüllter Wellenleiter ausgebildet sein; dadurch sind, anders als bei einem planaren Kondensator, voluminösere Strukturen möglich, die eine größere NOx- Menge aufnehmen können. Zudem ist der Einfluß des Speichermaterials auf die zu messende elektrische bzw. elektromagnetische Größe verstärkt, da bei höherer Frequenz gemessen werden kann.

Ein Kondensator ist vorzugsweise für niedrigere Frequenzen geeignet; wenn die Plattenausmaße in die Größenordnung der Wellenlänge der elektromagnetischen Wellen kommen, können die Signale nicht mehr einfach interpretiert werden.

### Zeichnung

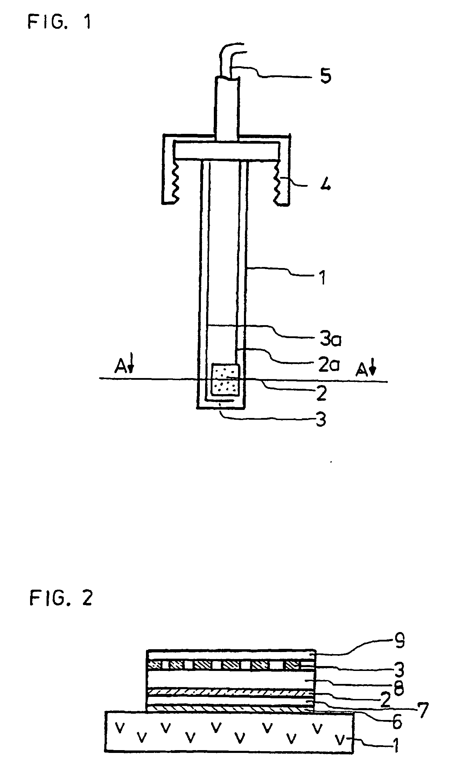

Ein Ausführungsbeispiel der Erfindung ist in der Zeichnung vereinfacht dargestellt und in der nachfolgenden Beschreibung näher erläutert. Es zeigen Figur 1 eine vereinfachte Darstellung eines erfindungsgemäßen Sensors und Figur 2 einen Querschnitt durch den Elektrodenbereich.

### Beschreibung der Ausführungsbeispiele

In der Figur 1 und 2 ist als Ausführungsbeispiel ein Sensor dargestellt in Form einer planaren Sonde, ähnlich der für die Messung des Luft/Kraftstoffverhältnisses bekannten Lambdasonde. Die Sonde ist in einen nicht dargestellten NOx- Speicherkatalysator eingebaut.

Auf einem planaren Keramikträger 1 aus vorzugsweise Aluminiumoxid sind in übereinanderliegenden Schichten ein Heizer 6 mit Abdeckung 7, eine Bodenelektrode 2 mit Zuleitung 2a, eine Schicht 8, die das Speichermaterial des NOx- Katalysators enthält sowie eine Deckelektrode 3 mit Zuleitung 3a aufgebracht. Es ist jedoch auch möglich, einen Sensor ohne Heizer einzusetzen.

Für die Elektroden werden übliche Elektrodenstoffe, z.B. Platin oder Platinmetalle verwendet. Die Deckelektrode besteht aus porösen Körnern, die leitfähig miteinander verbunden sind und den Zugang des Abgases zum Speichermaterial 8 ermöglichen.

Als Speichermaterial werden übliche NOx-Speicherstoffe eingesetzt, wie beispielsweise auf einem Träger aufgebrachte Alkalimetalle, Erdalkalimetalle, Seltenerdmetalle und/oder Edelmetalle.

Die Sensor kann eine poröse Abdeckung 9 als mechanische Schutzschicht und/oder als katalytische Schicht tragen.

Über ein Zuleitungskabel 5 werden die elektrischen Anschlüsse für Elektroden und Heizer geführt. Mit der Überwurfmutter 4 wird die Sonde im nicht dargestellten NOx- Katalysator befestigt.

Als Sensorprinzip wird die Änderung einer elektrischen oder elektromagnetischen Eigenschaft des Katalysator- Speichermaterials eingesetzt.

Wird der erfindungsgemäße Sensor in einen NOxhaltigen Gasstrom, z.B. in den Abgasstrom eines Mager- oder Dieselmotors gebracht, so wird das Speichermaterial des Katalysators zunehmend mit NOx beladen.

Gleichzeitig ändert sich mit zunehmendem Gehalt an polaren NOx-Molekülen beispielsweise die Dielektrizitätskonstante des Speichermaterials und somit die Kapazität eines Kondensators, dessen Dielektrikum das Speichermaterial ist. Auf diese Weise kann der aktuelle Beladungsgrad des Speichermaterials gemessen werden. Ist die Speicherkapazität des Katalysators erschöpft, wird über eine geeignete Vorrichtung ein Fettimpuls erzeugt und damit die NOx- Desorption in bekannter Weise bewirkt.

Der erfindungsgemäße Sensor kann über einen Heizer 6 in einen geeigneten Temperaturbereich gebracht werden, wo keine Anlagerung von Wasser oder von anderen Komponenten erfolgt, so daß die Querempfindlichkeit verbessert wird.

Durch geeignete Wahl einer Abdeckschicht lassen sich eventuell notwendige Vorreaktionen katalysieren und ein mechanischer Schutz sicherstellen.

Erfindungsgemäß können jedoch auch andere Eigenschaften des Katalysator- Speichermaterials, die sich mit der adsorbierten NOx- Menge ändern, als Grundlage für den Sensor dienen, beispielsweise dielektrische Verluste, Permeabilität, magnetische Verluste oder Widerstandsbelag. Diese Parameter können besonders gut in einem Resonator, Hohlraumresonator oder einem gefüllten Wellenleiter gemessen werden. Dabei kann der Katalysator selbst vollständig oder teilweise oder als separater und in mit dem Original-Katalysator identischer oder modifizierter Form in eine der obengenannten Sensorstrukturen eingesetzt werden. Die genannten Sensoren basieren auf einem gemeinsamen Prinzip, der Schwächung eines elektromagnetischen Feldes durch Beschlagen des Speichermaterials mit den polaren NOx- Molekülen.

Auch kann durch Messung der Adsorption elektromagnetischer Strahlung durch die Moleküle des adsorbierten Gases, besonders der Verbindungen dieses Gases mit dem Speichermaterial, der Beladungszustand nachgewiesen werden.

## Patentansprüche

1. Sensor zur Überwachung eines NOx-Katalysators, der ein Speichermaterial (8) zur Speicherung von NOx mittels Adsorption aufweist, wobei das Speichermaterial (8) das sensitive Element des Sensors bildet.

2. Sensor nach Anspruch 1, **dadurch gekennzeichnet, daß** der Sensor auf eine elektrische oder elektromagnetische Eigenschaft des Speichermaterials anspricht, die sich mit der adsorbierten NOx- Menge ändert.

3. Sensor nach Anspruch 2, **dadurch gekennzeichnet, daß** die elektrische Eigenschaft die Dielektrizitätskonstante ist.

4. Sensor nach Anspruch 3, **dadurch gekennzeichnet, daß** der Sensor als Kondensator ausgebildet ist.

5. Sensor nach Anspruch 2, **dadurch gekennzeichnet, daß** die elektromagnetische Eigenschaft die dielektrischen Verluste, die Permeabilität oder die magnetischen Verluste sind.

6. Sensor nach Anspruch 2, **dadurch gekennzeichnet, daß** der Sensor als Resonator, Hohlraumresonator oder als gefüllter Wellenleiter ausgebildet ist.

7. Sensor nach Anspruch 2, **dadurch gekennzeichnet, daß** die elektromagnetische Eigenschaft die Absorption der elektromagnetischen Strahlung ist.

8. Sensor nach Anspruch 1, **dadurch gekennzeichnet, daß** der Sensor in den Katalysator integriert ist.

9. Sensor nach Anspruch 1, **dadurch gekennzeichnet, daß** das sensitive Element Alkalimetalle, Erdalkalimetalle, Seltenerdmetalle und/oder Edelmetalle enthält.

## Claims

1. Sensor for monitoring an NOx catalytic converter which includes a storage material (8) for storing NOx by means of adsorption, the storage material (8) forming the sensitive element as the sensor.

2. Sensor according to Claim 1, **characterized in that** the sensor responds to an electrical or electromagnetic property of the storage material which changes with the amount of NOx adsorbed.

3. Sensor according to Claim 2, **characterized in that** the electrical property is the dielectric constant.

4. Sensor according to Claim 3, **characterized in that** the sensor is designed as a capacitor.

5. Sensor according to Claim 2, **characterized in that** the electromagnetic property is the dielectric losses, the permeability or the magnetic losses.

6. Sensor according to Claim 2, **characterized in that** the sensor is designed as a resonator, a cavity resonator or as a filled waveguide.

7. Sensor according to Claim 2, **characterized in that** the electromagnetic property is the absorption of the electromagnetic radiation.

8. Sensor according to Claim 1, **characterized in that** the sensor is integrated in the catalytic converter.

9. Sensor according to Claim 1, **characterized in that** the sensitive element contains alkali metals, alkaline-earth metals, rare earths and/or precious metals.

## Revendications

1. Capteur pour la surveillance d'un catalyseur de réduction des NOx, qui présente un matériau d'accumulation (8) pour accumuler les NOx par adsorption, dans lequel le matériau d'accumulation (8) forme l'élément sensible du capteur.

2. Capteur selon la revendication 1,
**caractérisé en ce que**
le capteur réagit sur une propriété électrique ou électromagnétique du matériau d'accumulation, qui change avec la quantité de NOx adsorbée.

3. Capteur selon la revendication 2,
**caractérisé en ce que**
la propriété électrique est la constante diélectrique.

4. Capteur selon la revendication 3,
**caractérisé en ce que**
le capteur est sous la forme d'un condensateur.

5. Capteur selon la revendication 2,
**caractérisé en ce que**
la propriété électromagnétique est les pertes diélectriques, la perméabilité ou les pertes magnétiques.

6. Capteur selon la revendication 2,
**caractérisé en ce que**
le capteur est sous la forme d'un résonateur, d'un résonateur à cavité ou d'un guide d'ondes à noyau.

7. Capteur selon la revendication 2,
**caractérisé en ce que**
la propriété électromagnétique est l'absorption du rayonnement électromagnétique.

8. Capteur selon la revendication 1,
**caractérisé en ce que**
le capteur est intégré dans le catalyseur.

9. Capteur selon la revendication 1,
**caractérisé en ce que**
l'élément sensible contient des métaux alcalins, des métaux alcalino-terreux, des métaux terres rares et/ou des métaux précieux.
